Europäisches Patentamt

European Patent Office

Office européen des brevets

(11) Publication number: **0 140 998**
**B1**

(12) **EUROPEAN PATENT SPECIFICATION**

(45) Date of publication of patent specification: **15.11.89**

(21) Application number: **83306676.4**

(22) Date of filing: **02.11.83**

(51) Int. Cl.⁴: **A 61 K 35/22** // A61K35/14

(54) **Ophthalmic preparations.**

(43) Date of publication of application:
**15.05.85 Bulletin 85/20**

(45) Publication of the grant of the patent:
**15.11.89 Bulletin 89/46**

(84) Designated Contracting States:
**CH DE FR GB IT LI**

(56) References cited:
**R. BASERGA: "Handbook of Experimental
Pharmacology", 1st edition, vol. 57, "Tissue
Growth factors", 1982, chapter 3, G.
CARPENTER: "Epidermal growth factor", pages
88-132, Springer, Berlin, DE
Exp. Eye Res. (1979) 28, pp147-157
Arch. Klin. exp. Ophthal. 210, pp 159-165 (1979)**

(73) Proprietor: **J C R KABUSHIKI KAISHA also
known as J C R INCORPORATED
148-3, Uchidekasuga-cho
Ashiya Hyogo 659 (JP)**

(72) Inventor: **Asaoka, Makoto
5-19, Hirosawa 1-chome
Hamamatsu Shizuoka 432 (JP)**
Inventor: **Hiratani, Hajime
705-3, Tottori Hannan-cho
Sennan-gun Osaka 599-02 (JP)**

(74) Representative: **Burford, Anthony Frederick
et al
W.H. Beck, Greener & Co. 7 Stone Buildings
Lincoln's Inn
London WC2A 3SZ (GB)**

Courier Press, Leamington Spa, England.

## Description

This invention relates to an ophthalmic preparation containing human epidermal growth factor (hereinafter referred to as h-EGF) as an active component.

More particularly, the invention relates to an ophthalmic preparation which is of value in the treatment of keratitis, corneal erosion, corneal infiltration and corneal ulcer.

h-EGF, which is derived from human urine, is a straight-chain polypeptide having 3 disulfide bridges and a molecular weight of about 6,000. This substance has epitheliogenic activity.

Regarding the action mechanism of h-EGF it has been suggested that the substance first binds itself to the EGF receptor on the cell membrane, then finds its way into the cell to induce DNA synthesis and, hence, proliferation of the cell [Journal of the Japan Medical Association *85*, (7) 833, 1981].

As to the tissue distribution of h-EGF, it occurs in the saliva, blood and urine at physiologically effective levels but does not occur in any appreciable amount in the lacrimal fluid, i.e. tears.

In local administration of h-EGF to patients with corneal injury, the present inventors found that the injuries were cured very soon after the treatment.

On the other hand, animal experiments and a trial in healthy subjects, which will hereinafter be quoted, revealed that the intact corneal cell is not influenced by the action of h-EGF.

Daniele et al (Albrecht v Graefes Arch. Klin, exp. Ophthal. (1979) *210*, 159—165) proposed mouse epidermal growth factor (hereinafter referred to as m-EGF) as a new and efficacious agent for increasing the restorative process of the corneal epithelium in many nondystrophic diseases. In particular, they reported acceleration of epithelial heating in vivo in human patients by administration of a saline solution containing 2 mg m-EGF/ml. Two drops of the solution were instilled every hour for five hours and subsequently at four hourly intervals until the cornea has healed.

Gospodarowicz and Greenburg (Exp. Eye. Res. (1979) *28*, 147—157) reported m-EGF accelerated the repair process of wounded endothelium of bovine, cat and human corneas in vitro. No details are provided of the experiments with cat or human corneas but in the experiments with bovine corneas 1 µg/ml m-EGF was used in Dulbecco's modified Eagle's tissue culture medium supplemented with 1% calf serum. They predicted that m-EGF also would accelerate the repair process in vivo.

Both Daniele et al and Gospodarowicz and Greenburg are acknowledged in R. Baserga: "Handbook of Experimental Pharmacology, 1st Edition, vol. 57, "Tissue Growth Factors" 1982, Chapter 3, G. Carpenter: "Epidermal Growth Factor", pages 88—132, Springer, Berlin.

To the best of our knowledge and belief, there has been no previous proposal to use h-EGF instead of m-EGF in the treatment of corneal injury. Further, the concentration employed in the compositions of this invention (0.01 to 50 µg/ml) are orders below the 2 mg/ml m-EGF concentrations taught by Daniele et al.

After administration of h-EGF to mice and rats in groups of 6 individuals of either sex at the dose level of 10 mg/kg by the subcutaneous route and at the dose of 1 mg/kg by the intravenous route (the doses equivalent to about 1,000,000 and about 100,000 times the amount in the blood of human/kg) caused no change in general condition, or death, indicating that it would not produce any toxic effects. Another experiment in which a 5 µg/ml solution of h-EGF was administered to male and female rabbits in groups of 3 individuals of either sex at the dose of 3 drops per instillation at the frequency of 3 times daily for 1 month showed that it caused no abnormalities in the ocular membrane. It was therefore found that h-EGF is a polypeptide which has only a very low toxicity.

This invention, which has thus been based on the above findings, is directed to an ophthalmic preparation which comprises an effective amount of human epidermal growth factor and an ophthalmologically acceptable diluent.

The ophthalmic preparation of h-EGF can be made available in such dosage form as an ophthalmic lotion or an ophthalmic ointment. The concentration of h-EGF in such an ophthalmic preparation is in the range of 0.01 to 50 µg/ml (or g).

In said ophthalmic lotion, water or an aqueous buffer solution may be used as a diluent.

The buffer used for pH adjustment in the preparation of this invention may be any buffer substance that is opthalmologically acceptable.

Thus, for example, such a combination as sodium chloride, monopotassium phosphate, potassium chloride, calcium chloride and magnesium sulfate; sodium dihydrogen phosphate and disodium hydrogen phosphate; or sodium chloride, potassium chloride and calcium chloride may be used.

For the preparation of said ophthalmic ointment, a diluent (base) such as white vaseline, purified lanoline and liquid paraffin may be employed.

To prepare the ophthalmic ointment, h-EGF may for instance be directly mixed with such a base or alternatively first dissolved in water, then mixed with lanolin and finally mixed with a further amount of lanolin or another ointment base. In addition to these procedures, any other established pharmaceutical procedure for the preparation of ophthalmic ointments may be employed, if desired.

Furthermore, in the ophthalmic preparation according to this invention, there may also be incorporated any of such conventional preservatives as chlorobutanol, methyl p-oxybenzoate and benzyl alcohol in addition to said pH-adjusting buffers.

The pH of the ophthalmic preparation need only within the ophthalmologically acceptable range of pH 5.0 to 8.0 and the preparation may be prepared by subjecting the composition to sterilization by millipore filtration within the pH range. h-EGF is stable in the above pH range.

The following Examples and Test Example are further illustrative of the preparation of this invention.

Example 1

In sterile pure water were dissolved 0.835 w/v % of sodium chloride, 0.007 w/v % of monopotassium phosphate, 0.022 w/v % of potassium chloride, 0.017 w/v % of calcium chloride, 0.030 w/v % of magnesium sulfate. Then, h-EGF was added to portions of this solution to prepare liquids containing h-EGF in the concentrations of 0.5 µg/ml, 1.5 µg/ml and 4.5 µg/ml, respectively. Finally, 0.15% of chlorobutanol was added to the above liquids and the entire compositions were subjected to millipore filtration to give ophthalmic lotions.

Example 2

In sterile distilled water were dissolved 0.825 w/v % of sodium chloride, 0.145 w/v % of potassium chloride and 0.015 w/v % of calcium chloride. Then, h-EGF was added to portions of the above solution in the concentration of 10 µg/ml, 15 µg/ml and 30 µg/ml, respectively followed by bacterial filtration to give ophthalmic lotions.

Example 3

In a small amount of pure water was dissolved 100 g of h-EGF and the solution was thoroughly kneaded with 10 g of purified lanolin and, then, with a further 100 g of white lanolin to give an ophthalmic ointment.

Test Example

(1) The 10 µg/ml, 15 µg/ml and 30 µg/ml ophthalmic lotions prepared in Example 2 were administered to male and female rabbits in groups of 3 individuals of either sex in the dose of 3 drops per instillation at the frequency of 3 times daily for 1 month to investigate the influence of the drugs on the ocular membrane. There were no abnormal findings.

(2) Using 2 rabbits, uniform corneal injuries were produced in both eyes of each rabbit with an electric iron and the 1.5 µg/ml ophthalmic lotion of Example 1 was instilled into one eye and physiological saline into the fellow eye in dose of 3 to 4 drops at the frequency of 3 times daily to investigate the healing process. The results are shown in Table 1.

TABLE 1

| | Time to cure, in days | |
|---|---|---|
| Rabbit No. | H-EGF 1.5 µg/ml | Saline |
| 1 | 3 days | 9 days |
| 2 | 4 days | 10 days |

(3) To healthy subjects, the 0.5 µg/ml, 1.5 µg/ml and 4.5 µg/ml ophthalmic lotions of Example 1 were administered in the dose of 3 to 4 at the frequency of 4 times daily for 1 week. There were no abnormal findings.

Example 4

The 1.5 µg/ml ophthalmic lotion of Example 1 was administered to patients with keratitis, corneal erosion, corneal infiltration or corneal ulcer in the dose of 3 to 4 drops per instillation at the frequency of 4 times daily. The results are shown in Table 2. The time to complete cure was as short as about 1/2 of the time period hitherto experienced, showing that the drug produces an early cure, especially in cases of corneal trauma due to contact lenses.

TABLE 2

| Disease | No. of effec-tive cases | Time to complete cure (in days) |
|---|---|---|
| Keratitis | 18/20 | 2—6 days |
| Corneal erosion | 20/20 | 1—5 days |
| Corneal infiltration | 15/15 | 4—5 days |
| Corneal ulcer | 8/15 | 7—14 days |

Six cases sampled randomly from among the above cases, will be described below in further detail.

Case 1: A 34-year-old male.
Right eye. Corneal erosion (corneal burn).
Hot water entered into the right eye and the epithelium of the entire cornea was thermally ablated. At visit, pain (+++), ciliary hyperemia (+++), and visual acuity 0.01. The patient was instructed to instill 3 to 4 drops of a 1.5 µg/ml h-EGF ophthalmic lotion into the eye four times daily. On the following day, the visual acuity was restored. Pain and ciliary hyperemia were improved to (+) and (−), respectively. The topical application of the drug was continued for one additional day. When he visited the hospital on the next day, the cornea was quite clear and the subjective symptoms had disappeared.

Case 2. A 32-year-old male
Left eye. Corneal erosion
A synthetic resin adhesive agent (cyanoacrylate) having entered into his left eye, the patient visited the hospital. Immediately, foreign bodies were eliminated. The elipthelium of the entire cornea had become detached; pain (+++), ciliary hypermia (++), and visual acuity 0.06. The instillation of a 1.5 µg/ml h-EGF ophthalmic lotion was instituted (3 to 4 drops, four times daily).
On the following day, pain was slightly improved (++) but epithelial ablation and ciliary hyperemia (++) remained unchanged.
On the fourth day, ersoion became very slight but pain (+) and ciliary hyperemia (++) persisted. The patient was instructed to continue the topical application of the h-EGF ophthalmic lotion, and on the sixth day, the cornea became quite clear and the subjective symptoms completely disappeared. Visual acuity recovered to 1.0.

Case 3. A 82-year-old female
Left eye. Corneal ulcer
Ulcer occurred in the area of previous parenchymatous keratitis, resulting in pain (++), ciliary hyperemia (++) and severe visual disorder. Instillation of a 1.5 µg/ml h-EGF ophthalmic lotion was instituted. The dosage was 3 to 4 drops four times daily.
On the fourth day, pain and ciliary hyperemia improved to (+) and (±), respectively.
On the seventh day, pain and ciliary hyperemia both improved to (−), but slight ulcer persisted in the central area.
In the eleventh day, the cornea was stained only very faintly on fluorescein staining. By the fifteenth day, the corneal ulcer had completely healed and the visual acuity recovered to 30 cm/n.d.

Case 4. A 61-year old male
Right eye. Corneal infiltration
The patient visited the hospital because of onset of moderate corneal infiltration. Pain (+), ciliary hyperemia (++), and visual acuity 0.5.
Topical therapy with 3 to 4 drops of a 1.5 µg/ml h-EGF ophthalmic lotion four times daily was instituted.
Consequently, by the seventh day, the infiltration had subsided almost completely and the subjective symptoms disappeared, and the instillation of the drug was discontinued. However, 5 days later, infiltration relapsed and the h-EGF ophthalmic therapy was reinstituted according to the same dosage regimen. On the 10th day after reinstitution both the infiltration and subjective symptoms disappeared. No recurrence was found thereafter. Visual acuity was 0.5.

Case 5. A 67-year-old male
Both eyes. Keratitis
Keratitis occurred in the surface stratum of corneal macula, of which the patient had a past history. Pain (+), ciliary hyperemia (+), and visual acuity 0.09, right and 0.03, left.
Three to four drops of the 1.5 µg/ml h-EGF ophthalmic lotion were instilled into the eye four times daily. By the fourth day, both the corneal inflammation and subjective symptoms had almost disappeared. On the sixth day, the keratitis was completely cured. The visual acuity was 0.2, right and 0.09, left.

Case 6. A 38-year old male
Right eye. Corneal ulcer
Corneal ulcer developed around the pseudopterygium. Pain (+), photophobia (++), ciliary hyperemia (++), foreign body sensation (++), and visual acuity 1.2. h-EGF ophthalmic lotion, 1.5 μg/ml, (3 to 4 drops four times daily) was topically applied. On the third day, the epithelium began to cover the ulcerative area and the only subjective symptom was foreign body sensation.
On the tenth day, the cornea ceased to be stained with fluorescein and the subjective symptom disappeared. Visual acuity=1.5.

## Claims

1. An ophthalmic preparation characterised in that it comprises human epidermal growth factor at a concentration of 0.01 to 50 μg/ml (or g).
2. A preparation as claimed in Claim 1, wherein said concentration is 0.5 to 30 μg/ml (or g).
3. A preparation as claimed in Claim 2, wherein the said concentration is 0.5 to 4.5 μg/ml (or g).
4. A preparation as claimed in any one of the preceding claims, wherein the preparation is an ophthalmic lotion.
5. A preparation as claimed in Claim 4, wherein human epidermal growth factor is diluted with an aqueous buffer solution.
6. A preparation as claimed in any one of Claims 1 to 3, wherein the preparation is an ophthalmic ointment.
7. A preparation as claimed in Claim 6, wherein human epidermal growth factor is diluted with vaseline, lanoline or liquid paraffin.
8. A preparation as claimed in Claim 6 or Claim 7, wherein the preparation contains 10 μg/ml of human epidermal growth factor.
9. The use of human epidermal growth factor in the manufacture of an ophthalmic preparation for use in the treatment of keratitis, corneal erosion, corneal infiltration or corneal ulcer, characterised in that the human growth factor is present in a concentration of 0.01 to 50 μg/ml (or g).

## Patentansprüche

1. Ophthalmologisches Präparat, dadurch gekennzeichnet, daß es einen menschlichen epidermalen Wachstumsfaktor in einer Konzentration von 0,01 bis 50 μg/ml (oder g) umfaßt.
2. Präparat nach Anspruch 1, wobei die Konzentration des Wachstumsfaktors 0,5 bis 30 μg/ml (oder g) beträgt.
3. Präparat nach Anspruch 2, wobei die Konzentration des Wachstumsfaktors 0,5 bis 4,5 μg/ml (oder g) beträgt.
4. Präparat nach einem der vorhergehenden Ansprüche, wobei das Präparat als ophthalmologische Lotion vorliegt.
5. Präparat nach Anspruch 4, wobei der menschliche epidermale Wachstumsfaktor mit einer wäßrigen Pufferlösung verdünnt ist.
6. Präparat nach einem der Ansprüche 1 bis 3, wobei das Präparat also ophthalmologische Salbe vorliegt.
7. Präparat nach Anspruch 6, wobei der menschliche epidermale Wachstumsfaktor, mit Vaseline, Lanolin oder flüssigem Paraffin verdünnt ist.
8. Präparat nach Anspruch 6 oder 7, wobei 10 μg/ml des Wachstumsfaktors für die menschliche Epidermis darin enthalten sind.
9. Verwendung des menschlichen epidermalen Wachstumsfaktors zur Herstellung eines ophthalmologischen Präparats zur Verwendung bei der Behandlung von Keratitis, Hornhauterosion, Hornhautinfiltration oder Hornhautgeschwür, dadurch gekennzeichnet, daß der menschliche epidermale Wachstumsfaktor in einer Konzentration von 0,01 bis 50 μg/ml (oder g) vorhanden ist.

## Revendications

1. Préparation ophtalmique caractérisée en ce qu'elle comprend un facteur de croissance épidermique humain à une concentration de 0,01 à 50 μg/ml (ou g).
2. Préparation selon la revendication 1, caractérisée en ce que ladite concentration est de 0,5 à 30 μg/ml (ou g).
3. Préparation selon la revendication 2, caractérisée en ce que ladite concentration est de 0,5 à 4,5 μg/ml (ou g).
4. Préparation selon l'une des revendications précédentes, caractérisée en ce que ladite préparation est une lotion ophtalmique.

5. Préparation selon la revendication 4, caractérisée en ce que le facteur de croissance épidermique humain est dilué avec une solution tampon aqueuse.

6. Préparation selon l'une des revendications 1 à 3, caractérisée en ce que ladite préparation est une pommade ophtalmique.

7. Préparation selon la revendication 6, caractérisée en ce que le facteur de croissance épidermique humain est dilué avec de la vaseline, de la lanoline ou de la paraffine liquide.

8. Préparation selon la revendication 6 ou la revendication 7, caractérisée en ce que ladite préparation contient 10 µg/ml de facteur de croissance épidermique humain.

9. Utilisation du facteur de croissance épidermique humain dans la fabrication d'une préparation ophtalmique pour l'emploi dans le traitement de kératite, érosion cornéenne, infiltration cornéenne ou ulcère cornéen, caractérisée en ce que le facteur de croissance épidermique humain est présent à une concentration de 0,01 à 50 µg/ml (ou g).